Europäisches Patentamt

(19) European Patent Office

Office européen des brevets

(11) Publication number: **0 160 673**
**B1**

(12) **EUROPEAN PATENT SPECIFICATION**

(45) Date of publication of patent specification: **11.01.89**

(21) Application number: **84903894.8**

(22) Date of filing: **29.10.84**

(86) International application number:
**PCT/NO84/00046**

(87) International publication number:
**WO 85/02016 09.05.85 Gazette 85/11**

(51) Int. Cl.⁴: **G 01 N 27/22, G 01 N 33/28**

(54) **AN APPARATUS FOR THE MEASUREMENT OF THE FRACTION OF GAS IN A TWO-COMPONENT FLUID FLOW COMPRISING A LIQUID AND A GAS IN MIXTURE.**

(30) Priority: **02.11.83 NO 833983**

(43) Date of publication of application:
**13.11.85 Bulletin 85/46**

(45) Publication of the grant of the patent:
**11.01.89 Bulletin 89/02**

(84) Designated Contracting States:
**BE DE FR GB NL SE**

(56) References cited:
**EP-A-0 080 632**
**DE-A-2 160 526**
**DE-A-3 227 631**
**DE-B-1 910 217**
**SE-B- 411 593**

**Derwent's abstract no. 94547 D/51 SU 813234**

(73) Proprietor: **DEN NORSKE STATS OLJESELSKAP A.S.**
**Postboks 300 Forus**
**N-4001 Stavanger (NO)**

(72) Inventor: **Thorn, Richard**
**104 Riverside Drive**
**Fulham 5024 S.A. (AU)**

(74) Representative: **Rees, David Christopher et al**
**Kilburn & Strode 30 John Street**
**London WC1N 2DD (GB)**

Courier Press, Leamington Spa, England.

## Description

This invention relates to an apparatus for measuring the fraction of gas in a two component fluid flow comprising a liquid and a gas in mixture, in particular a flowing oil/gas mixture.

The fraction of gas in a two component flow of liquid and gas may be defined as the volume of gas in an arbitrary section of a tube divided by the volume of that section of the tube. The fraction of gas may therefore be expressed as a number varying from zero (when the flow is entirely comprised by the liquid) to one (when the flow solely consist of gas).

While it has been of great importance to know how much of the production from an oil well is comprised by oil and how much of it is gas, measurement of the fraction of gas has always been considered with great interest within the offshore oil business.

Measuring devices whose operating principle is based on the detection of changes in capacitance, have been increasingly employed for the measurement of the fraction of gas in a two-component fluid comprising liquid/gas mixtures.

The principle for such a measuring method is well known and amounts essentially to measuring the electrical capacitance across two electrodes, between which the mixture of the two components is flowing. If the area and the mutual separation of the electrodes are fixed, the measured capacitance will be related to the fraction of gas in the mixture between the electrodes.

Derwent abstract 94547 D/51 of Russian patent SU813—234 (Karataev) and European patent application EP—A1—00800632 (Nissan) both disclose arrangements for measurements on oil by the use of capacitive elements and methods.

Derwent abstract 94547 discloses a gas/liquid stream analyser comprising a vessel which separates a two-component fluid in a measuring section of pipe into a gas collection chamber and a liquid collection chamber. Capacitive pickups associated with each chamber detect when each chamber is full. The two readings are then compared with a third capacitive reading of the fluid in the measuring section. A reading is thus derived of the percentage of each component.

EP—A1—0080632 discloses an apparatus for detecting the degree of deterioration of lubricating oil in an internal combustion engine. A sensor comprises a pair of spaced electrodes. The sensor is connected to a fixed capacitor to form a voltage divider network excited by an AC voltage source. As the dielectric constant of the oil changes the AC voltage reading will also change in proportion.

By employing such techniques, it is in principle possible to build instruments with rapid dynamic response. The instrument may be constructed in such a way as to give a non-intrusive method of measurement.

In spite of the advantages which are achievable, commercially available gas fraction meters based on the capacitance principle are relatively scarce.

Russian Inventor's Certificate No. 587383 discloses a capacitive converter comprising a pair of planar electrodes mounted on an enclosing resilient tubular electrode at zero potential. The tubular electrode is supported on a dielectric base. When a mixture passed through an aperture in the base, the temperature changes. The temperature change expands or contracts the dielectric base which distorts the tubular electrode changing the distance between the planar electrodes. Thus, the capacitive converter is self compensating.

Thus, apparatus for the measurement of the fraction of gas in a two-component flow comprising a liquid and a gas as a mixture, for example, a flowing oil gas mixture, is known which is arranged to measure alterations of the electrical capacitance across two electrodes working at mutually different potentials, between which the two component mixture is forced to flow, which electrodes form an integral part of a primary sensor, the apparatus also comprising a third tubular-shaped electrode which encloses the two electrodes and a signal processing unit, including a sinewave voltage generator, arranged to apply a potential difference between the pair of electrodes.

The main disadvantage of known capacitance gas fraction meters is the dependence of the instrument calibration upon the nature of the flow regime being monitored.

Thus, for example, the calibration curve required for a bubble flow will deviate from that of a stratified flow.

One of the reasons for this flow regime dependency is the missing homogeneity of the electric field through the sample volume. If the electric field varies through the whole sample volume, the measured change in capacitance which arises from a change in the gas fraction, will depend upon where the gas is located.

The object of the present invention is to remove these disadvantages and to provide a gas fraction meter of the above mentioned kind in which a homogeneous electric field is maintained within the sample-volume of the sensor. In addition it is also an object of the present invention provide a non-intrusive measuring device of sturdy and simple construction, by which an output voltage proportional to the fraction of gas is generated by means of a simple signal processing circuit.

The present invention is characterised by the features as claimed by Claim 1.

Besides being capable of fulfilling the above mentioned requirements, experiments have shown the gas fraction measuring apparatus of the present invention not to be as dependent on the flow regime as known gas fraction measuring devices comprising only two electrodes.

The measuring device according to the invention will be explained in more detail in the following with reference to the drawing, in which:

Figure 1 shows a gas fraction meter comprising two main components, namely a primary sensor, shown schematically, and a signal processing unit illustrated as a circuit diagram; and

Figure 2 illustrates the distribution of the electrical field inside the primary sensor.

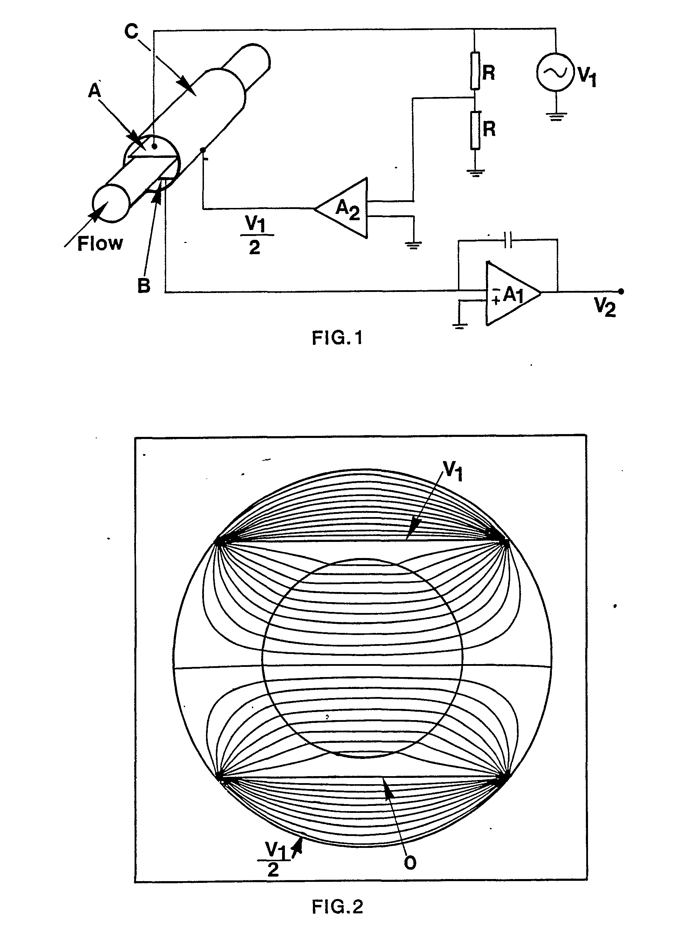

As shown in Figure 1, the primary sensor comprises three electrodes, namely two plate electrodes A and B in parallel together with a third electrode C, which, shaped like a tube, encloses the other two electrodes A, B. The two component flow, for example in the form of an oil/gas mixture, which is going to be monitored, passes through between the parallel plate electrodes A, B and thereby caused alterations in the measured capacitance between A and B.

This capacitance is measured by means of a sine wave generator and an amplifier $A_1$ with capacitance feedback; better known today by the term "charge amplifier". Because the input of this amplifier is kept clamped on "virtual earth", any alteration in the leakage capacitance to each will have very little influence on the result. This makes it possible to employ long screened cables between sensor and amplifier (if this is necessary), without reducing the accuracy of the measurement in any substantial degree. If the feedback capacitance of the charge amplifier $A_1$ is fixed and the amplitude $V_1$ of the sinewave generator is kept constant, the amplitude $V_2$ of the output signal from the amplifier will be directly proportional to the alterations in the capacitance of the primary sensor. The output voltage of the charge amplifier is in other words a measure of the fraction of gas in the mixture being monitored.

In order to maintain a homogeneous electric field within the sample volume of the primary sensor, the electrode C must be kept at a potential equivalent to the potential occurring half way between the electrodes A and B. This can be accomplished by using a simple voltage divider and a buffer amplifier $A_2$.

Figure 2 illustrates the lines of equipotential inside the primary sensor, where the electrode A is kept at a potential $V_1$, the electrode B at a potential equal to 0 and the electrode C accordingly at a potential equal to $V_1/2$. As shown by the field curves in Figure 2, the lines of equipotential are homogeneous inside the sample region of the primary sensor.

## Claims

1. Apparatus for the measurement of the fraction of gas in a two-component fluid flow comprising a liquid and a gas as a mixture, for example a flowing oil/gas mixture, the apparatus being arranged to measure alterations of the electrical capacitance between a pair of planar electrodes (A, B), working at mutually different potentials, between which the two-component mixture is forced to flow, which electrodes (A, B) form an integral part of a primary sensor, the apparatus also comprising a third tubular-shaped electrode (C) which encloses the two electrodes (A, B) and a signal processing unit, including a sinewave voltage generator, arranged to apply a potential difference between the pair of electrodes (A, B), characterised in that the third electrode (C) is arranged to produce a homogeneous electric field within the sample volume of the sensor, and in that means ($A_2$) are provided for maintaining the third electrode at a potential ($V_1/2$) at least approximately corresponding to the arithmetic mean of the potentials applied to each of the pair of electrodes (A, B), the signal processing unit further comprising means ($A_1$) arranged to derive a signal related to the capacitance between the electrodes (A, B).

2. Apparatus as claimed in claim 1 characterised in that the means for maintaining the third electrode (C) at a potential at least approximately corresponding to the difference between the potentials applied to each of the pair of electrodes (A, B), comprises a voltage divider and a buffer amplifier ($A_2$) connected with the output of the sinewave voltage generator.

3. Apparatus as claimed in claim 1 or 2 characterised in that the means arranged to derive a signal related to the capacitance between the electrodes (A, B) comprises a charge amplifier ($A_1$).

4. Apparatus as claimed in claim 3 characterised in that the charge amplifier is an operational amplifier ($A_1$) with capacitive feedback coupled to one of the pair of electrodes (B), and in that the third electrode (C) is connected to a voltage divider across the sinewave generator.

## Patentansprüche

1. Vorrichtung zur Messung der Gasfraktion in einem Zweikomponenten-Fluidstrom, umfassend eine Flüssigkeit und eine Gas als Mischung, beispielsweise -eine strömende Öl-/Gas-Mischung, wobei die Vorrichtung zum Messen von Änderungen der elektrischen Kapazität zwischen einem Paar bei gegenseitig unterschiedlichen Spannungspotentialen arbeitenden ebenen Elektroden (A, B), ausgebildet ist, zwischen denen die Zweikomponentenmischung zwangsweise hindurchströmt und die einen integralen Teil eines Primärsensors bilden, wobei die Vorrichtung weiter eine dritte, rohrförmige Elektrode (C), die die zwei Elektroden (A, B) umschließt, und eine Signalverarbeitungseinheit umfaßt, die einen Sinuswellen-Spannungsgenerator, der zum Anlegen einer Potentialdifferenz zwischen dem Elektrodenpaar (A, B) vorgesehen ist, umfaßt, dadurch gekennzeichnet, daß die dritte Elektrode (C) zum Erzeugen eines homogenen elektrischen Feldes in dem Meßvolumen des Sensors angeordnet ist und daß Mittel ($A_2$) zum Halten der dritten Elektrode auf einem Potential ($V_1/2$) vorgesehen sind, das mindestens ungefähr dem arithmetischen Mittel der an jede Elektrode des Elektrodenpaares (A, B), angelegten Potentiale entspricht, wobei die Signalverarbeitungseinheit weiterhin Mittel ($A_1$) umfaßt, die zum Ableiten eines auf die Kapazität zwischen den Elektroden (A, B) bezogenen Signals vorgesehen sind.

2. Vorrichtung nach Anspruch 1, dadurch gekennzeichnet, daß die Mittel zum Halten der dritten Elektrode (C) auf einem mindestens ungefähr der Differenz zwischen den an jede Elektrode des Elektrodenpaares (A, B) angelegten Potentials/entsprechenden Potential einen Spannungsteiler und einen Trennverstärker ($A_2$) umfassen, die an den Ausgang des Sinuswellen-Spannungsgenerators geschaltet sind.

3. Vorrichtung nach Anspruch 1 oder 2, dadurch gekennzeichnet, daß die zum Ableiten eines auf die Kapazität zwischen den Elektroden (A, B), bezogenen Signals vorgesehenen Mittel einen Ladeverstärker ($A_1$) umfassen.

4. Vorrichtung nach Anspruch 3, dadurch gekennzeichnet, daß der Ladeverstärker ein Operationsverstärker ($A_1$) mit kapazitiver Rückkopplung ist, die mit einer Elektrode (B) des Elektrodenpaares gekoppelt ist, und daß die dritte Elektrode (C) an einen zum Sinuswellen-Generator parallelen Spannungsteiler geschaltet ist.

**Revendications**

1. Appareil de mesure de la proportion de gaz dans un écoulement de fluide à deux composants comprenant un mélange de liquide et de gaz, par exemple un mélange d'écoulement huile/gaz, cet appareil étant agencé pour mesurer les modifications de la capacité électrique entre une paire d'électrodes planes (A, B), fonctionnant à des potentiels mutuellement différents, entre lesquelles le mélange à deux composants est obligué de s'écouler, lesquelles électrodes (A, B) font partie intégrante d'un détecteur primaire, cet appareil comprenant également une troisième électrode de forme tubulaire (C) qui entoure des deux électrodes (A, B) et un module de traitement de signal, comprenant un générateur de tension sinusoidale, agencé pour appliquer une différence de potentiel entre la paire d'électrodes (A, B), caractérisé en ce que la troisième électrode (C) est agencée pour produire un champ électrique homogène dans le volume d'échantillon du détecteur, et en ce que des moyens ($A_2$) sont prévus pour maintenir la troisième électrode à un potentiel ($V_1/2$) correspondant au moins approximativement à la moyenne arithmétique des potentiels appliquès à chacune de la paire d'électrodes (A, B), le module de traitement de signal comprenant en outre des moyens ($A_1$) agencés pour fournir un signal lié à la capacitance entre les électrodes (A, B).

2. Appareil selon la revendication 1, caractérisé en ce que les moyens pour maintenir la troisième électrode (C) à un potentiel au moins approximativement égal à la différence entre les potentiels appliqués à chacune de la paire d'électrode (A, B) comprennent un diviseur de tension et un amplificateur tampon ($A_2$) connecté à la sortie du générateur de tension sinusoidale.

3. Appareil selon l'une des revendications 1 ou 2, caractérisé en ce que les moyens agencés pour fournir un signal associé à la capacitance entre les électrodes (A, B) comprennent un amplificateur de charge ($A_1$).

4. Appareil selon la revendication 3, caractérisé en ce que l'amplificateur de charge est un amplificateur opérationnel ($A_1$) avec une réaction capacitive appliquée à l'une des deux électrodes (B) et en ce que la troisième électrode (C) est connectée à un diviseur de tension aux bornes du générateur de tension sinusoidale.

EP 0 160 673 B1

FIG.1

FIG.2